# EUROPEAN PATENT APPLICATION

(11) **EP 1 946 729 A1**
(43) Date of publication of application: **23.07.2008**
(21) Application number: 07001977.3
(22) Date of filing: 30.01.2007
(51) Int. Cl.: A61F 5/56

(54) **Apparatus for preventing snoring and method using same**

(30) Priority: 17.01.2007 KR 20070005210
(71) Applicant: Bio Sleep Med Co., Ltd., Seongbuk-gu Seoul (KR)
(72) Inventor: Hong, Junghwa, Dongan-gu Anyang-si Gyeonggi-do (KR); Shin, Chol, Seocho-gu Seoul (KR)
(74) Representative: Samson & Partner

(57) **Abstract**

An apparatus for preventing snoring includes a human body wearable unit to be worn by a sleeper; air chambers provided at a rear of the human body wearable unit, the air chambers being capable of shrinking and expanding; an air supply unit for supplying air to the air chambers; and a sound detecting unit having installed at the human body wearable unit for sensing a sound generated by the sleeper and for generating the sound as an electrical signal. The apparatus further includes a controller, when a snoring of the sleeper is sensed, for controlling the air supply unit to supply an air to the air chambers, after receiving the signal outputted from the sound detecting unit, wherein a posture of the sleeper is changed by the air chambers having the air supplied from the air supply unit.

## Description

The present invention relates to an apparatus for preventing snoring and a snoring prevention method using same; and, more particularly, to an apparatus for preventing snoring and a snoring prevention method using same, which is capable of unaffectedly changing a sleep posture to stop the snoring without awakening the sleeper and capable of making such a posture change accurately even if a sleeper moves unconsciously by accurately determining whether or not the sleeper snores based on an ambient noise.

In general, a habitual snoring, an obstructive sleep apnea and an upper airway resistance syndrome classified as a sleep-disordered breathing are diseases in which the repetitive closure of the upper airway occurs during sleep. Such diseases hinder sound sleep by deteriorating sleep efficiency at night and especially decrease a blood oxygen saturation rate [see Chrokroverty S. (1994) Sleep Disorder Medicine. Butterworth-Heinemann].

The sleep-disordered breathing causes a daytime drowsiness, a deteriorated power of concentration, a memory loss, a decreased learning ability, a chronic fatigue and the like. Further, the sleep-disordered breathing leads to accidents at construction sites and workplaces and traffic accidents caused by a drowsy driving, thereby inflicting social and economical damages.

There have recently been several reports on a close relationship between the sleep-disordered breathing and the occurrence of obesity, high blood pressure, diabetes, dementia, cardiovascular diseases, cardiac paralysis, sexual function decline, cerebrovascular diseases, paralysis and metabolic syndrome [see Prospective study of the association between sleep-disordered breathing and hypertension. N Engl Med 2000; 342: 1378-1384].

An upper airway closure of a human body like this will be explained in the following with reference to the accompanying drawings.

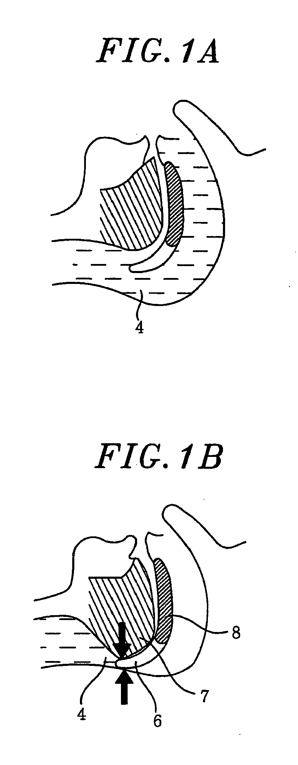

As shown in Fig. 1A, an upper airway 4 for introducing air into a bronchus and a lung (all not shown) is sufficiently secured in a normal state. However, referring to an obstructive sleep apnea state illustrated in Fig. 1B, a soft tissue 6 extended from a back part of a palate 8 is pressed by a self-weight and a weight of a tongue 7, thereby closing the upper airway 4.

Sleeping in a supine position worsens the closure of the upper airway 4. Further, such closure of the upper airway 4 leads to an obstructive sleep apnea in which breathing stops or is disrupted during sleep, and snoring occurs when the upper airway 4 is partially closed during sleep.

Various methods have been attempted to treat the snoring or the obstructive sleep apnea.

In general, there have been attempts of treating the sleep-disordered breathing by use of a functional pillow sheet during sleep, and there have been developed pillow sheets which prevent the sleep-disordered breathing through a posture correction by mainly elevating a lateral position or a head position.

Recently, a memory foam pillow sheet made of polymer foam developed by NASA has been commercially sold while claming as the pillow sheet for preventing a sleep-disordered breathing. Although such a memory foam pillow sheet is ergonomically designed to absorb load and shock transmitted to a human body and return to an original shape after the load is released, it does not bring a significant improvement for the treatment of a snoring or an obstructive sleep apnea.

Many apparatuses and methods have been developed for solving such problems, and this will be explained with reference to the accompanying drawings in the following.

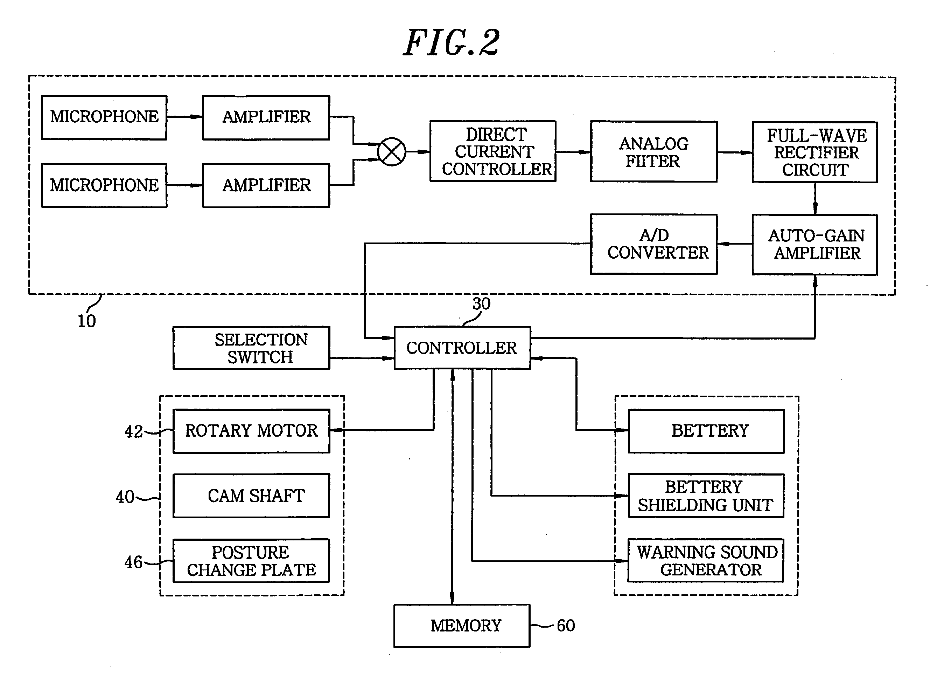

In accordance with an embodiment of a prior art, a utility model No. 20-0236225 which is filed with and registered in the Korean Intellectual Property Office, as shown in Fig. 2 is an "apparatus of driving a snoring prevention pillow sheet by sensing snoring." The snoring prevention apparatus includes a snoring signal sensing unit 10 which inputs snoring and various other noises and outputs a desired snoring signal; a controller 30 which analyzes the signal applied from the snoring signal sensing unit 10 and starts a rotary motor driver 40 if the applied signal is confirmed as a snoring signal; a memory which stores the signal applied from the controller 30 or outputs to the controller 30; and a rotary memory driver 40 where a rotary motor 42 is driven by the signal applied from the controller 30 to drive a posture change plate 46, thereby changing the shape of a pillow sheet. The controller 30 processes the snoring signal and the noise signal applied from the snoring signal sensing unit 10 to obtain a final data and calculate a time duration when the final data continuously has a value which is larger than a threshold value. The controller 30 calculates the number of maximum points which exist for the duration if the duration is higher when compared with a set-time. The controller 30 determines the signal as the snoring signal if the number of maximum points is higher than a set value for maximum points. Further, the controller 30 applies drive signals to the rotary motor driver 40 if the snoring signal is sensed as many times as the number of the set value to drive the rotary motor 42.

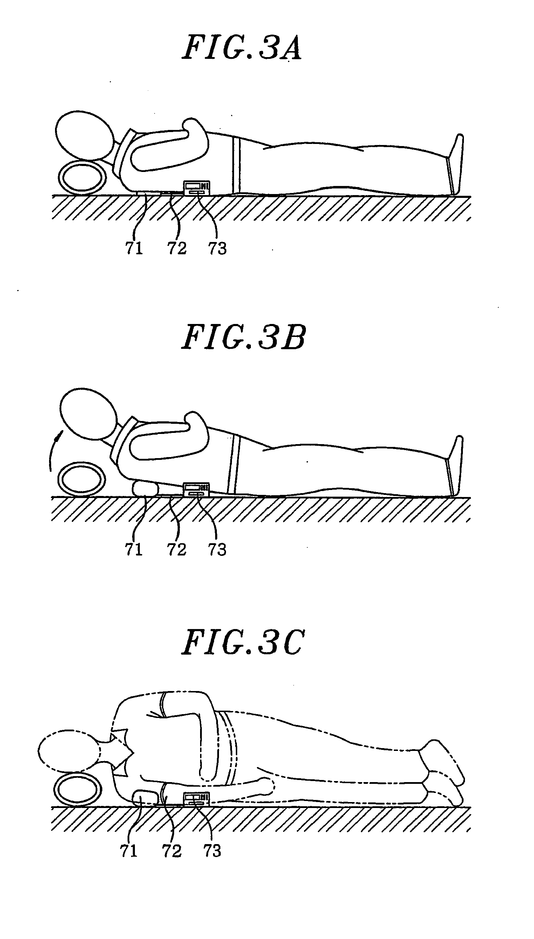

In accordance with another embodiment of the prior art, a utility model No. 20-0395890, which is filed for and registered in the Korean Intellectual Property Office, as shown in Figs. 3A to 3C is an "apparatus for reducing snoring." The snoring prevention apparatus includes an air pad 71 which detects the breath of a sleeper and is expanded by the air injected thereinto when snoring is sensed, thereby correcting the sleep posture of the sleeper; an air tube 72 which is combined with the air pad 71 to transmit the detected breathing to the next stage and to act as a passage of injecting and discharging air; a sensor for converting the breathing transmitted to the air tube 72 into an electrical signal; a preamplifier which pre-amplifies the breathing sense signal outputted from the sensor; a band pass filter unit which filters the breathing sense signal outputted from the preamplifier for each band and outputs it as a heart beat/ breathing/ snoring determining signal; a function input unit for inputting function and mode conversion signals; a central processing unit 73 which determines whether or not there is snoring based on the signal obtained by the preamplifier and the band pass filter unit in response to the signal inputted from the function input unit, which controls to display a breathing state, generates an air injection signal for expanding the air pad if it is determined as snoring, and generates a control signal to discharge the air injected into the air pad after the set time if no snoring is sensed after the air pad is expanded; a display which displays on a screen the breathing state, the heart beat status and the like of the sleeper in accordance with the state display control signal transmitted from the central processing unit 73; a driver for outputting a light emitting diode control signal, the air injection control signal and a discharge control signal, which are outputted from the central processing unit, as each corresponding drive signal for each of the air injection control and the discharge control signal; a light emitting diode (LED) for visually displaying whether or not power is supplied and a function operation state in accordance with a light emitting diode drive signal outputted from the driver; a motor for driving a pump which injects air to the air pad 71 in accordance with the air injection signal outputted from the driver; and a discharge solenoid which opens and closes a discharge valve in accordance with the discharge control signal outputted from the driver.

In this way, the snoring prevention apparatus in accordance with the embodiment of the prior art in Fig. 2 automatically drives a snoring prevention pillow sheet to incline the sleeper to one side if snoring is sensed and determined, thereby enabling the sleeper not to snore. However, the snoring prevention apparatus according to the embodiment of the prior art cannot return to the original state from a state in which the snoring prevention pillow is inclined after sensing a snoring, unless the user manually resets the apparatus. There is a problem in operating such an apparatus reliably in that it becomes hard to prevent snoring if the sleeper moves away from the pillow sheet through movement or position change during sleep. Further, the sleeper's sound sleep might be disturbed because noise is generated due to a mechanical friction between a cam shaft and a plate.

Further, the snoring prevention apparatus according to another embodiment of the prior art in Figs. 3A to 3C induces posture change by use of the air pad 71, but is not an active type and could awake the sleeper by disturbing the sound sleep of the sleeper because it is a type which encourages to change himself/herself his/her postures. There also lies a problem in operating such an apparatus reliably because, in case that the sleeper moves away from the expansion portion of the air pad 71 when the sleeper moves or changes his position, even such a posture change cannot be induced.

It is, therefore, a primary object of the present invention to provide an apparatus for preventing snoring and method thereof, which is capable of unaffectedly changing a sleep posture to stop the snoring without awakening the sleeper and capable of making such a posture change accurately even if the sleeper is made to move unconsciously by accurately determining whether or not the sleeper snores based on an ambient noise, thereby enabling the sleeper to carry on a sound sleep.

In accordance with an aspect of the present invention, there is provided an apparatus for preventing snoring, including:
a human body wearable unit to be worn by a sleeper;
air chambers provided at a rear of the human body wearable unit, the air chambers being capable of shrinking and expanding;
an air supply unit for supplying air to the air chambers;
a sound detecting unit having installed at the human body wearable unit for sensing a sound generated by the sleeper and for generating the sound as an electrical signal; and
a controller, when a snoring of the sleeper is sensed, for controlling the air supply unit to supply an air to the air chambers, after receiving the signal outputted from the sound detecting unit, in order to change a posture of the sleeper when a snoring of the sleeper is sensed.

In accordance with another aspect of the present invention, there is provided a method of preventing snoring in which a sleeper wears a human body wearable unit where an shrinkable and expandable air chambers are provided at the rear of the human body wearable unit, the method comprising the steps of:
measuring the sound generated by the sleeper (S10),
determining whether or not there is a snoring of the sleeper based on the measured sound (S20), and
supplying air to the air chamber to expand the air chambers when a snoring is present in the sound, to change a posture of the sleeper.

The above and other objects and features of the present invention will become apparent from the following description of embodiments, given in conjunction with the accompanying drawings, in which:
Figs. 1A and 1B are drawings explaining a closure of an upper airway of human body;
Fig. 2 is a drawing illustrating a main part of a conventional snoring prevention apparatus;
Figs. 3A to 3C are drawings illustrating another conventional snoring prevention apparatus;
Figs. 4A and 4B show a front and a rear view of a snoring prevention apparatus in accordance with an embodiment of the present invention;
Fig. 5 depicts a configuration diagram of a snoring prevention apparatus in accordance with the embodiment of the present invention;
Fig. 6 is a flow chart illustrating a snoring prevention method according to the present invention; and
Figs. 7A to 7C are drawings for explaining the operation of the snoring prevention apparatus according to the present invention.

Hereinafter, embodiments of the present invention will be described in detail with reference to the accompanying drawings.

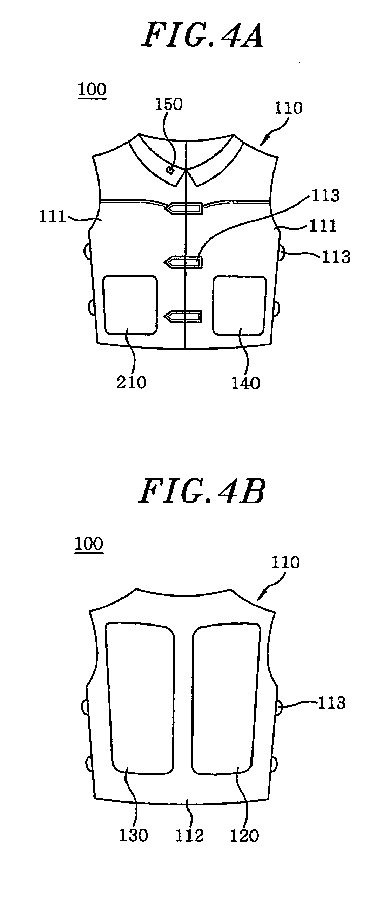

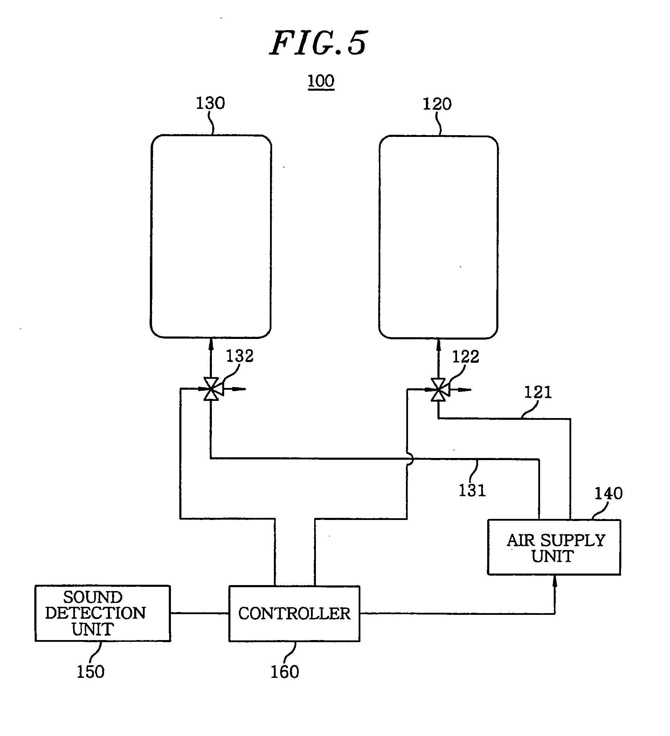

Figs. 4A and 4B show a front and a rear view of a snoring prevention apparatus in accordance with an embodiment of the present invention, and Fig. 5 shows a configuration diagram of a snoring prevention apparatus in accordance with the embodiment of the present invention. As shown above, a snoring prevention apparatus 100 of the present invention includes a human body wearable unit 110 which to be worn by a sleeper; an air chambers 120, 130 provided at the rear of the human body wearable unit 110; an air supply unit 140 for supplying air to the air chambers 120, 130; a sound detecting unit 150 for detecting the sound generated by the sleeper; and a controller 160 which receives the signal outputted from the sound detecting unit 150 and controls the air supply unit 140.

The human body wearable unit 110 to be worn by the sleeper adopts a design of a wearable unit while considering wearability and usability from a number of different design angles. Further, a functional structure and a suitable material selection are essential in such a wearable design. In particular, a material for regulating human body from harmful factors such as pleasantness, stability, electromagnetic wave shielding function, static electricity prevention function, insulation function and the like, are controlled can be selected.

In order to carry out a temperature control function, the human body wearable unit 110 not only discharges the sweat excreted during sleep through the material, but is also configured to have air holes in all directions of the body or in the armpit region where sweat can come out easily, so that the heat or humidity within the clothes can be discharged quickly, thereby improving the wear comfort. And, it is necessary to consider a sewing method of enabling for minimizing the friction between the material and the human body. In this regard, the human body wearable unit 110 may be made of health oriented materials (e.g., chitosan fiber, silver fiber, bamboo fiber or the like, hi-tech materials such as aqua-trans, cool max mesh or the like), or environment friendly materials (e.g., organic cotton, tencel, natural mineral ion health fiber or the like).

The human body wearable unit 110, though not shown in drawings, can be configured in a winter jacket or a jumper form. Considering that it is to be worn during sleep, it may be configured in a vest shape as shown in Figs. 4A and 4B. Further, the human body wearable unit 110 may be formed in a sleeveless vest form to make it better in permeability compared to a vest having sleeves, which in turn restrain factors increasing body temperature during sleep.

The human body wearable unit 110 can be made in various vest shapes such as a shape of having only front adjustment, a shape of having front adjustment and side open, a shape of having neither adjustment nor open, or the like. However, the human body wearable unit 110 might be made in a shape of having front adjustment and side open in this embodiment, and includes a pair of front portions 111 which covers the upper front surface of the sleeper and a rear portion 112 which covers the upper rear surface of the sleeper. The front adjustment between the front portions 111 and the side open between the front part 111 and the rear portion 112 are connected to be separable by easy-to-use fasteners 113 with which gap adjustment is easy.

The fastener 113 of the human wearable unit 110 is only an example, and various other combining and disjoining units other than the fastener such as zippers, rubber bands, snap buttons and the like can be used in conjunction with the same or a different kind, in order to connect the front adjustment and the side open.

Since the human body wearable unit 110 can be worn even when the front adjustment and the side open are opened, wearing and taking off the unit 110 are made easy even for patients having restricted body movement. Further, air ventilation is possible through the front adjustment and side open, thus the factors that increase the body temperature during sleep are greatly restrained. In addition, the human body wearable unit 110 can be worn for any body sizes and possibly minimize pressure working against body during sleep, which in turn aids the sleeper to have a sound sleep.

The air chambers 120, 130 are provided at the rear of the human body wearable unit 110, i.e., at the rear portion 112 and they can be shrunk or expanded. The air chambers 120, 130 are expanded by air supplied from the air supply unit 140 and are made in a structure or/and of a material which can be shrunk and expanded. In such a structure, the air chambers 120, 130 may include a part which can be sheared or darted. Further, for such a material, the air chambers 120, 130 might be employ, e.g., synthetic resin, natural rubber, artificial rubber, fiber and the like, which can be expanded or shrunk. The air chambers 120, 130 can have a sufficient volume to change the posture of the sleeper, e.g., from a supine position to a lateral position.

The air chambers 120, 130 are positioned to incline the sleeper to one side at the rear of the human body wearable unit 110, so as to easily and naturally change the sleeper posture from the supine position to the lateral position when expanded. Here, the air chambers 120, 130 can be provide in a pair as illustrated in the embodiment of the present invention so as to alternately change the posture of the sleeper from the supine position to the lateral position of left side or right side.

The air supply unit 140 selectively supplies air to the air chambers 120, 130 via air supply lines 121, 131 in accordance with the control signal of the controller 160. In this regard, a compressed gas can be used, and an air pump may be used to supply air through pumping.

The sound detecting unit 150 is a sound measuring sensor such as a kind of microphone and the like and is installed in the human body wearable unit 110. The sound detecting unit 150 is installed to be adjacent to the respiratory organ such as the mouth or the nose of the sleeper so that the snoring signal generated by the sleeper can be accurately detected while comparing with the ambient noise during sleep. To this end, the sound detecting unit 150 can be installed in a collar 114 of the human body wearable unit 110 or around the neck of the human body in case the human body wearable unit 110 does not include such a collar 114.

The controller 160 receives the signal outputted from the sound detecting unit 150 and rectifies the voltage measured by the sound detecting unit 150 to do envelope detection, and the snoring is sensed when a value over a fixed threshold is observed. When sensing the snoring, the air supply unit 140 is configured to supply air to the air chamber in order to change the posture of the sleeper, e.g., from the supine position to the lateral position, which in turn opens the upper airway of the sleeper solving the disordered breathing of the sleeper.

On other hand, in case that the air chambers 120, 130 are provided in a pair, as shown in this embodiment, the controller 160 receives the signal from the sound detecting unit 150 and alternately expands the air chambers 120, 130 to put the sleeper in a lateral position facing a direction different from a previous position each time the snoring is sensed.

The controller 160 can control the air supply unit 140 to directly shrink as well as expand the air chambers 120, 130. For example, if the air supply unit 140 is an air pump, the shrinkage and the expansion are achieved through a forward rotation and a reverse rotation by the pump. Further, although it is not limited thereto, the three-way valves 122, 132, as in the embodiment of the present invention, controlled by the controller 160 are installed on a route, i.e., the air supply line 121, 131, respectively, through which the air is supplied from the air supply unit 140, so that the expanded air chambers 120, 130 can discharge the air for shrinkage.

The three-way valves 122, 132 may be a solenoid valves which can be operated by control signals from the controller 160, for example.

On the other hand, the air supply unit 140, the controller 160 and the like can be installed to be separated from the human body wearable unit 110, but can also be separately attached thereto for the sake of a convenient use and in order to minimize the interference with the sleeper. Here, the controller 160 can be installed within a control box 210 (shown in Fig. 4A) of which the outer surface has a plurality of manipulation buttons (not shown) and stuck to the human body wearable unit 110, and the power supplied to the air supply unit 140, the controller 160 and the like is provided by an easy-to-use rechargeable battery.

The operation of the snoring prevention apparatus made in such a structure will be superseded with a detail explanation for the snoring prevention method in accordance with an embodiment of the present invention.

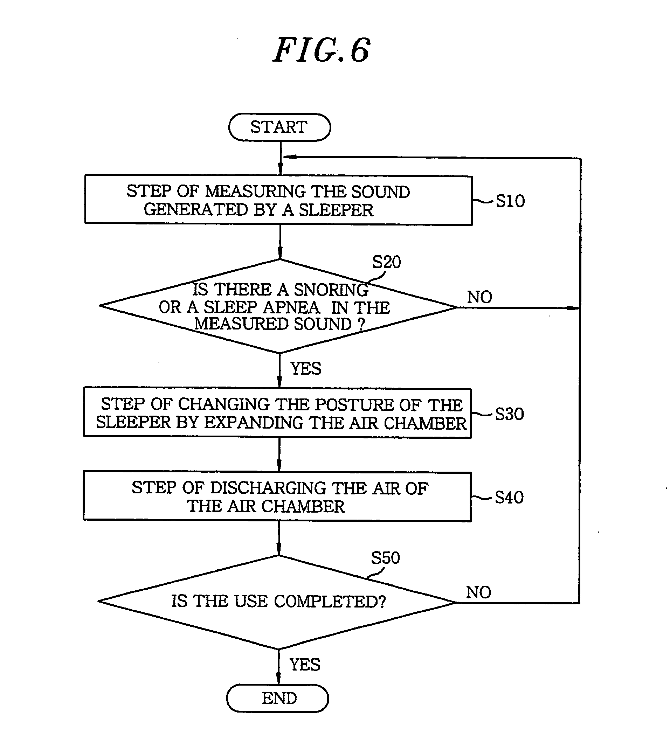

Fig. 6 is a flow chart illustrating a snoring prevention method in accordance with an embodiment of the present invention. As shown in the drawings, the snoring prevention method of the present invention is for preventing the snoring of the sleeper who wears the human body wearable unit 110 where there is provided the shrinkable and expandable air chamber at the rear thereof. The snoring prevention method includes of the steps of: measuring the sound generated by the sleeper (S10), determining whether or not there is a snoring of the sleeper based on the measured sound (S20), and changing a posture of the sleeper when snoring is detected (S30).

In the step of measuring the sound generated by the sleeper (S10), the ambient noise from the surroundings of the sleeper is measured by the sound detecting unit 150 such as the microphone and the like. Here, the sound detecting unit 150 is installed in the collar 114 of the human body wearable unit 110 or around the neck, thus the snoring signal generated by the sleeper can be detected more easily than that generated by the ambient noise during sleep.

In the step of determining whether there is a snoring of the sleeper in the measured sound (S20), the controller 160 receives the signal transmitted from the sound detecting unit 150 and rectifies the voltage measured by the sound detecting unit 150 for an envelope detection, and the snoring is determined to be present when a value over a fixed threshold is sensed.

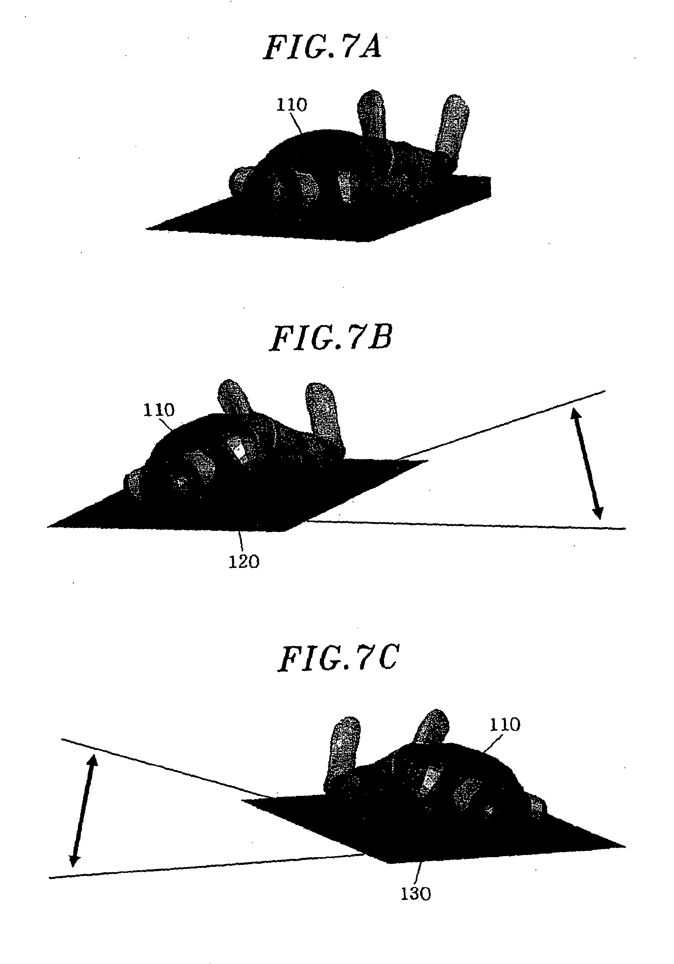

In the step of changing the posture of the sleeper (S30), the air supply unit 140 is operated when the controller 160 senses a snoring and supplies the air thereafter to the air chamber 120, thereby expanding the air chamber 120. Here, in case that the air chambers 120, 130 are provided in the left side and the right side of the rear of the human body wearable unit 110, respectively, as in the embodiment of the present invention, the air is first supplied to any one of the air chambers 120, 130, so as to change the posture of the sleeper from the supine position (shown in Fig. 7A) to the lateral position (shown in Fig. 7B), thereby opening the upper airway to thereby correcting disordered breathing of the sleeper.

In the step of changing the posture of the sleeper (S30), when a fixed time, e.g., between 5 minutes and 10 minutes, elapses after the posture of the sleeper is changed, the air of the air chamber 120 is discharged so that the posture of the sleeper can return to the normal state (S40). Here, the air discharge of the air chamber 120 can be made by the reverse operation of the air supply unit 140. However, in this embodiment, the discharge can be made through the three-way valve 122 which operates based on the control signal from the controller 160, in order to naturally bring the sleeper to the normal position through a smooth air discharge.

Meanwhile, if the fixed time elapses after the posture of the sleeper is changed, the sleeper returns to the supine position, as shown in Fig. 7A, by the air discharge of the air chamber, thereby offering a comfortable posture. Thereafter, whether or not the usage is completed is determined (S50), and if it is still in use, the steps following the step of measuring the sound generated by the sleeper (S10) are performed. Further, when the snoring is present, the air is supplied to the air chamber 130 different from the previous one, thereby changing the posture to the lateral position of the opposite direction, as shown in Fig. 7C. That is to say, in the step of changing the posture of the sleeper (S30), the air chambers 120, 130 located at a left and a right side of the rear of the human body wearable unit 110, respectively, are alternately expanded to achieve a different lateral position for the sleeper each time the snoring is detected.

In the step of changing the posture of the sleeper (S30), unlike the embodiment of the present invention, in case a single air chamber is provided to incline the sleeper to one side at the rear of the human body wearable unit 110, the sleeper is changed from the supine position to the lateral position by expanding the single air chamber.

According to the preferable embodiment of the present embodiment as above, the sound generated by the sleeper is measured by the sound detecting unit 150 to accurately determine whether or not the sleeper snores, and if snoring is detected, the sleep posture is naturally changed from the supine position to the lateral position by selectively expanding the air chambers 120, 130 so as to stop the snoring without awakening the sleeper unlike the prior art, thereby keeping the sleeper to have a sound sleep.

Further, the human body wearable unit 110 having the air chambers 120, 130 provided at the rear thereof is worn by the sleeper, thus the operation capable of changing the posture of the sleeper for preventing the snoring is highly reliable even though there is a change in location and posture during sleep. Further, the expanded air chambers 120, 130 are shrunk to its original state, to thereby enable the sleeper to return to the comfortable posture. In addition, the air chambers 120, 130 are operated by the supply of the air, thereby restraining the noise generated upon the operation thereof to minimize the sleep disturbance.

As described above, the snoring prevention apparatus and the snoring prevention method thereof in accordance with the embodiment of the present invention can be configured to measure sound generated by the sleeper to accurately determine whether or not the sleeper snores, and naturally change the sleep posture of the sleeper, if the sleeper snores, by expanding the air chambers so as to stop snoring without awakening the sleeper. In this manner, the sleeper can be kept to have a sound sleep. Further, the human body wearable unit 110 having the air chambers provided at the rear thereof is worn by the sleeper, thus the operation for preventing the snoring is highly reliable even though the position or the posture is changed during sleep. Further, the expanded air chamber is shrunk to the original state, thus the sleeper can return to a comfortable posture while the noise generated upon the operation is restrained to minimize the sleep disturbance.

While the invention has been shown and described with respect to the preferred embodiments, it will be understood by those skilled in the art that various changes and modification may be made without departing from the scope of the invention as defined in the following claims.

## Claims

1. An apparatus for preventing snoring, comprising:
a human body wearable unit to be worn by a sleeper;
air chambers provided at a rear of the human body wearable unit, the air chambers being capable of shrinking and expanding;
an air supply unit for supplying air to the air chambers;
a sound detecting unit having installed at the human body wearable unit for sensing a sound generated by the sleeper and for generating the sound as an electrical signal; and
a controller, when a snoring of the sleeper is sensed, for controlling the air supply unit to supply an air to the air chambers, after receiving the signal outputted from the sound detecting unit, in order to change a posture of the sleeper when a snoring of the sleeper is sensed.

2. The apparatus of claim 1, wherein the human body wearable unit is formed in a vest shape.

3. The apparatus of claim 1, wherein one of the air chambers is positioned to incline the sleeper to one side at the rear of the human body wearable unit, in order to change the posture of the sleeper from a supine position to a lateral position upon expansion.

4. The apparatus of claim 1, wherein the air chambers include three-way valves controlled by the controller and installed on a route through which the air is supplied to discharge the air.

5. The apparatus of claim 1 or 4, wherein two of the air chambers are provided at a left and a right side of the rear of the human body wearable unit, respectively, and
the controller receives a signal from the sound detecting unit and alternately expands the air chambers so as to put the sleeper in a lateral position facing a direction different from a previous position each time the snoring is detected.

6. The apparatus of claim 1, wherein the sound detecting unit is provided at a collar of the human body wearable unit or around a neck.

7. A method of preventing snoring in which a sleeper wears a human body wearable unit where an shrinkable and expandable air chambers are provided at the rear of the human body wearable unit, the method comprising the steps of:
measuring the sound generated by the sleeper (S10),
determining whether or not there is a snoring of the sleeper based on the measured sound (S20), and
supplying air to the air chamber to expand the air chambers when a snoring is present in the sound, to change a posture of the sleeper.

8. The method of claim 7, wherein changing the posture of the sleeper is changing the posture of the sleeper from a supine position to a lateral position by an expansion of the air chambers provided to incline the sleeper to one side at a rear of the human body wearable unit.

9. The method of claim 7, wherein changing the posture of the sleeper is alternately expanding the air chambers which are located at a left and a right side of the rear of the human body wearable unit, respectively, so as to put the sleeper in a lateral position facing a direction different from a previous position each time the snoring is detected.

10. The method of claim 7, wherein changing the posture of the sleeper further comprises the step of:
discharging the air in the air chamber if a fixed time elapses after the posture of the sleeper is changed, to return the posture of the sleeper to an original position.

## Amended claims

### Amended claims in accordance with Rule 137(2) EPC.

**1.** An apparatus for preventing snoring, comprising:
a human body wearable unit to be worn by a sleeper;
air chambers provided at a rear of the human body wearable unit, the air chambers being capable of shrinking and expanding;
an air supply unit for supplying air to the air chambers;
a sound detecting unit having installed at the human body wearable unit for sensing a sound generated by the sleeper and for generating the sound as an electrical signal; and
a controller, when a snoring of the sleeper is sensed, for controlling the air supply unit to supply an air to the air chambers, after receiving the signal outputted from the sound detecting unit, in order to change a posture of the sleeper when a snoring of the sleeper is sensed.

**2.** The apparatus of claim 1, wherein the human body wearable unit is formed in a vest shape.

**3.** The apparatus of claim 1, wherein one of the air chambers is positioned to incline the sleeper to one side at the rear of the human body wearable unit, in order to change the posture of the sleeper from a supine position to a lateral position upon expansion.

**4.** The apparatus of claim 1, wherein the air chambers include three-way valves controlled by the controller and installed on a route through which the air is supplied to discharge the air.

**5.** The apparatus of claim 1 or 4, wherein two of the air chambers are provided at a left and a right side of the rear of the human body wearable unit, respectively, and
the controller receives a signal from the sound detecting unit and alternately expands the air chambers so as to put the sleeper in a lateral position facing a direction different from a previous position each time the snoring is detected.

**6.** The apparatus of claim 1, wherein the sound detecting unit is provided at a collar of the human body wearable unit or around a neck.
